(19) Europäisches Patentamt
European Patent Office
Office européen des brevets

(11) Veröffentlichungsnummer: **0 144 285**
**B1**

(12) **EUROPÄISCHE PATENTSCHRIFT**

(45) Veröffentlichungstag der Patentschrift: **26.10.88**

(51) Int. Cl.⁴: **C 07 D 493/22**, A 01 N 43/24, A 61 K 31/355 // (C07D493/22, 313:00, 311:00, 311:00, 307:00)

(21) Anmeldenummer: **84810519.3**

(22) Anmeldetag: **29.10.84**

(54) **Neue Lactone zur Bekämpfung von tierischen und pflanzlichen Schädlingen.**

(30) Priorität: **02.11.83 CH 5909/83**
**02.10.84 CH 4736/84**

(43) Veröffentlichungstag der Anmeldung: **12.06.85 Patentblatt 85/24**

(45) Bekanntmachung des Hinweises auf die Patenterteilung: **26.10.88 Patentblatt 88/43**

(84) Benannte Vertragsstaaten: **AT BE CH DE FR GB IT LI NL SE**

(56) Entgegenhaltungen:
**THE JOURNAL OF ANTIBIOTICS, Band XXXIII, Nr. 10, Oktober 1980, Seiten 1120-1127, Tokyo, JP; YO TAKIGUCHI et al.: "Milbemycins, a new family of macrolide antibiotics: Fermentation, isolation and physico-chemical properties"**

(73) Patentinhaber: **CIBA-GEIGY AG, Klybeckstrasse 141, CH-4002 Basel (CH)**

(72) Erfinder: **Gehret, Jean-Claude, Dr., Im Aeschfeld 12, CH-4147 Aesch (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft Milbemycin-Derivate der Formel I, Verfahren zu ihrer Herstellung durch Singulett-Sauerstoff-Oxidation und ihre Verwendung in der Schädlingsbekämpfung als solche oder in Form geeigneter Mittel. Verbindungen der Formel I

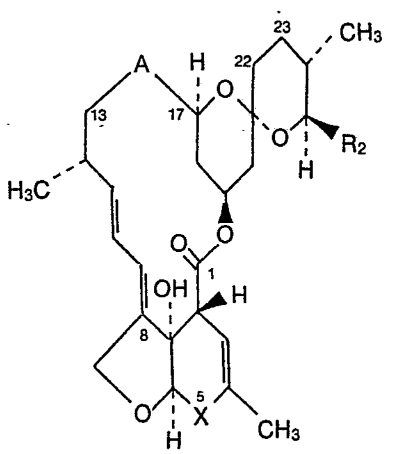

(I)

werden in zwei Reihen von Strukturen nebeneinander gewonnen. Bei Verbindungen der Formel Ia bedeutet

A die Struktur

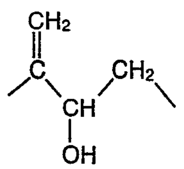

[$=\Delta^{14,29}$–15–ol] (Ia)

bei Verbindungen der Formel Ib bedeutet

A die Struktur

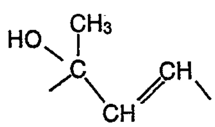

[=14-Hydroxi–$\Delta^{15,16}$] (Ib)

während in beiden Fällen X entweder

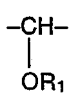 oder 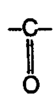 ist,

und $R_1$ Wasserstoff, eine Silylgruppe der Formel

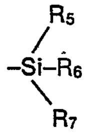

darstellt, worin $R_5$ einen $C_1$–$C_4$-aliphatischen Rest oder Benzyl und $R_6$ und $R_7$ unabhängig voneinander einen $C_1$–$C_4$-aliphatischen Rest, Benzyl oder Phenyl bedeuten oder für die Acylgruppen $R_3$–CO– oder $R_4$–$SO_2$– steht,

worin $R_3$ einen unsubstituierten oder halogenierten $C_1$–$C_6$-aliphatischen Rest oder unsubstituiertes oder durch $C_1$–$C_4$-Alkyl oder Halogen substituiertes Phenyl bedeutet und $R_4$ einen $C_1$–$C_4$-Alkylrest oder einen unsubstituierten oder durch Methyl, Chlor oder Nitro substituierten Phenylrest darstellt;

$R_2$ Methyl, Ethyl, Isopropyl oder sek.Butyl darstellen.

Verbindungen der Reihe Ia und Ib lassen sich durch physikochemische Methoden voneinander trennen.

Verbindungen, worin $R_2$ sek.Butyl darstellt, sollen hier und im folgenden gleichfalls zu den Milbemycin-Derivaten gerechnet werden, obwohl sie nach der üblichen Systematik nicht darunter fallen, sondern gemäss US-PS 4 173 571 von Avermectin-Derivaten abgeleitet sind.

Im Rahmen vorliegender Erfindung sind Verbindungen der Formel Ia bevorzugt, und unter ihnen solche, worin X wahlweise –CHOH– oder –CO– bedeutet. Acyl- und Silylgruppen des Substituenten $R_1$ sind im wesentlichen als Schutzgruppen aufzufassen, deren Anwesenheit aber den biologischen Wert der zugrundeliegenden Verbindungen mit $R_1$ = H nicht mindert.

Bevorzugt sind Verbindungen der Formel I, worin $R_1$ Wasserstoff, einen $R_3$–CO-Rest oder $R_4$–$SO_2$-Rest bedeutet, in welchen $R_3$ einen $C_1$–$C_4$-Alkylrest oder eine unsubstituierte oder durch Methyl oder Chlor substituierte Phenylgruppe darstellt und $R_4$ Methyl, Ethyl, Phenyl, p-Tolyl, o-Nitrophenyl, p-Chlorphenyl ist, während $R_2$ Methyl, Ethyl, Isopropyl oder sek.Butyl bedeutet.

Als Beispiele für $R_3$, die keine Limitierungen darstellen sollen, seien genannt Methyl, Ethyl, Propyl, Isopropyl, tert.Butyl, Phenyl, p-Chlorphenyl, p-Tolyl.

Eine bedeutende Gruppe von Verbindungen sind Milbemycin D-Derivate der Formel I, worin A die für Formel I genannten Bedeutungen hat, X –CHOH– oder –CO– darstellt und $R_2$ iso$C_3H_7$ ist.

Eine andere Gruppe bevorzugter Verbindungen der Formel Ia sind solche, worin $R_1$ die vorgenannte Silylgruppe darstellt, in der $R_5$ Methyl, Ethyl, Propyl, Isopropyl, tert.Butyl, und $R_6$ und $R_7$ unabhängig Methyl, Ethyl, Isopropyl, tert. Butyl, Phenyl oder Benzyl bedeuten, während $R_2$ Methyl, Ethyl, Isopropyl oder sek.Butyl ist.

Beispiele für Silyl-Gruppen sind Trimethylsilyl, Methyldiphenylsilyl, tris(tert.Butyl)silyl, Diphenyl-tert.butylsilyl, bis(Isopropyl)methylsilyl und besonders tert.Butyl-dimethylsilyl.

Die 13-Position ist in natürlich vorkommenden Milbemycinen ($R_1$ = H; $R_2$ = $CH_3$, $C_2H_5$ oder iso$C_3H_7$) unsubstituiert. Bei Avermectinen dagegen steht in der 13-Position ein β-Wasserstoff-Atom und ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen.

Durch Hydrolyse des Zucker-Restes der Avermectine gelangt man leicht zu den entsprechenden Avermectin-aglykonen, die eine allylische 13α-Hydroxy-Gruppe besitzen. Diese OH-Gruppe lässt sich durch Reaktion mit α-Nitrobenzolsulfonylchlorid in ein 13β-Chlor-Derivat überführen, dessen Chlor reduktiv mit tris(n-Butyl)zinnhydrid entfernt werden kann. Auf diese Weise ist die Überführung von Avermectin-Derivaten in die Milbemycin-Reihe möglich (Tetrahydron Letters, Vol. 24, No. 48, pp. 5333–5336 [1983]).

Verbindungen der Formel I werden erfindungsgemäss durch Singulett-Sauerstoff-Oxidation aus entsprechend substituierten Milbemycin-Derivaten [X = $-CH(OR_1)-$] oder 5-Keto-Milbemycin-Derivaten [X = $-CO-$] der Formel II

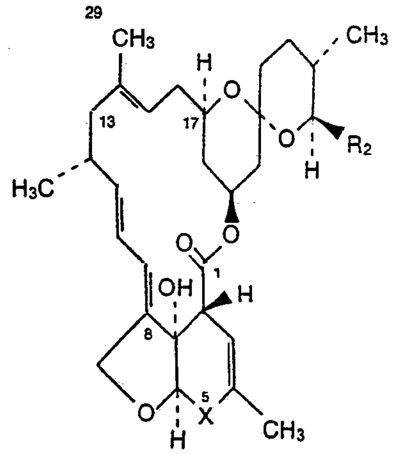

(II),

worin X und $R_2$ die für Formel I gegebene Bedeutung haben, und anschliessende selektive Reduktion des intermediär entstandenen 15-Peroxids und 14-Peroxids

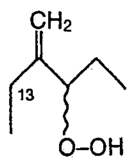

15-Peroxid

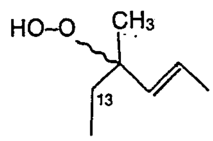

14-Peroxid

mit Natriumborhydrid, Lithiumaluminiumhydrid oder Triphenylphosphin erhalten. Die Reaktion wird bei sichtbarem Licht in Gegenwart eines Sensibilisators bei Normaldruck und bei einer Temperatur von −90 °C bis +45 °C, vorzugsweise 0 °C bis +29 °C, in einem inerten Lösungsmittel durchgeführt. Vorzugsweise wird in einer Bestrahlungsapparatur gearbeitet.

Der Reaktionsverlauf lässt sich schematisch so darstellen:

1) Sauerstoff + Licht + Sensibilisator
2) Reduktion

Verbindungen II ——→ Verb. I

(Vgl. H.H. Wassermann et al. «Singulett Oxygen»; Academic Press, New York 1979; oder B. Ranby et al. «Singulett Oxygen Reactions with Organic Compounds and Polymers», Wiley, New York 1978).

Als Lösungsmittel eignen sich z.B. Äther und ätherartige Verbindungen wie Diäthyläther, Diisopropyläther, Dioxan und Tetrahydrofuran; aromatische Kohlenwasserstoffe wie Benzol, Toluol und Xylole; Ketone wie Aceton, Methyläthylketon und Cyclohexanon; Nitrile wie Acetonitril; Ester wie Äthylacetat und Butylacetat; sowie Dimethylformamid, Dimethylsulfoxid und halogenierte Kohlenwasserstoffe, oder Mischungen dieser Lösungsmittel mit Wasser.

Als Sensibilisator eignen sich Farbstoffe wie z.B. Methylenblau, Bengalrosa, Chlorophyll, Erythrosin, Eosin, Zinktetraphenylporphin, Haematoporphyrin, Riboflavin, Fluorescein oder Acridinorange. Ohne weitere Aufarbeitung wird nach Abschluss der Oxydation bei einer Temperatur von 0° bis 20 °C selektiv reduziert.

Als Lichtquelle empfiehlt sich eine Lampe mit einer Leistung von 60–500 Watt, bevorzugt 100–350 Watt. Sofern Schutzgruppen für die 5-Hydroxi-Gruppe gewünscht werden, kommen die bereits für $R_1$ genannten Silyl- und Acyl-Gruppen in Frage oder z.B. ein Benzylether, Methoxiethoximethylether oder Dihydrofuran- oder Dihydropyran-Reste. Diese Schutzgruppen können in Verbindungen der Formel II eingeführt und später auf übliche Art wieder abgespalten werden.

Durch übliche Acylierung der 5-OH-Gruppe mit den entsprechenden Acylhalogeniden oder Acylanhydriden oder durch Reaktion der 5-OH-Gruppe mit dem entsprechend substituierten Silan-Derivat der Formel

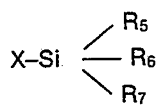

werden alle jene vorgenannten Derivate der Formel I oder II hergestellt, bei denen $R_1$ eine andere Bedeutung als Wasserstoff hat, und worin $R_5$, $R_6$ und $R_7$ die für die Formel I genannten Reste darstellen, wobei der Begriff Acylhalogenid für Acylchlorid oder Acylbromid steht und wobei X eine Silylabgangsgruppe bedeutet. Zu den Silylabgangsgruppen X zählen beispielsweise Bromid, Chlorid, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0°C bis 80°C, bevorzugt bei 10°C bis 40°C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage. Die Entfernung dieser Silyl- und Acylreste $R_1$ in der 5-Position geschieht durch selektive milde Hydrolyse (→ R = H) mit z.B. Arylsulfonsäure in alkoholischer Lösung oder nach einer anderen dem Fachmann geläufigen Methode.

Die Verbindungen der Formel II, worin $R_1$ Wasserstoff bedeutet, sind entweder aus der US-PS 3 950 360 bekannt geworden und wurden ursprünglich als «Antibiotika B-41-A», später als «Milbemycin-A»-Verbindungen bezeichnet, oder sind aus der US-PS 4 346 171 bekannt und werden als «B-41D» oder «Milbemycin-D» bezeichnet oder sind aus der US-PS 4 173 571 bekannt geworden und werden als 13-Deoxi-22,23-dihydro-Avermectin ($R_2$ = sec.Butyl) bezeichnet. Sie besitzen die Struktur

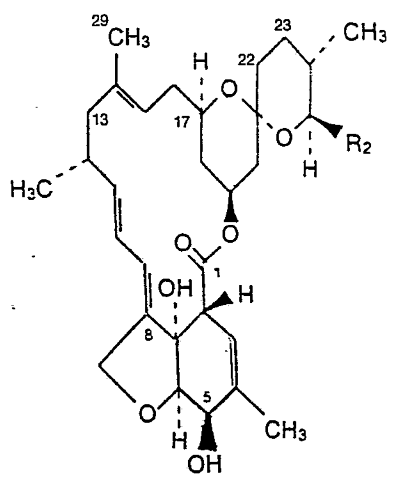

$R_2 = CH_3$ Milbemycin $A_3$
$R_2 = C_2H_5$ Milbemycin $A_4$
$R_2 = isoC_3H_7$ Milbemycin D
$R_2 = sec.C_4H_9$ 13-Deoxi-22,23-dihydro-C-076-Bla-aglycon, oder 13-Deoxi-22,23-dihydro-avermectin-Bla-aglykon.

Ein weiterer Gegenstand vorliegender Erfindung betrifft Schädlingsbekämpfungsmittel gegen Ekto- und Endoparasiten sowie Schadinsekten, die neben üblichen Trägerstoffen und/oder Verteilungsmitteln als mindestens einen Wirkstoff eine Verbindung der Formel I enthalten.

Die Verbindungen der Formeln I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haematopinidae); unter der Ordnung Diptera insbesondere Schädlinge der Familien Muscidae, Calliphoridae, Oesterridae, Tabanidae, Hippoboscidae, Gastrophilidae.

Die Verbindungen I sind auch einsetzbar gegen Hygiene-Schädlinge insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitärer Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnungen Homoptera, insbesondere gegen Schädlinge der Familien Aphididae, Delphacidae, Cicadellidae, Psyllidae, Loccidae, Diaspididae und Eriophyidae (z.B. die Rostmilbe auf Citrusfrüchten); der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizide gegen Schädlinge im Erdboden geeignet.

Die Verbindungen der Formeln I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen I sind auch wirksam gegen Pflanzen-Nematoden der Arten Meloidogyne, Heterodera, Pratylenchus, Ditylenchus, Radopholus, Rhizoglyphus und andere.

Darüberhinaus sind die Verbindungen I gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesphagostomum, Chabertia, Trichuris, Strongylus, Trichonema, Dictyocaulus, Cappillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Die Verbindungen I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend ausgewählt.

Die Verbindungen I werden bei Warmblütern in Aufwandmengen von 0,01 bis 50 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$–$C_{22}$), wie z.B. die Na- oder K-Salze der Öl- oder Stearinsäure, oder von natürlichen Fettsäuregemischen, die z.B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4–14)-Aethylenoxid-Adduktes oder Phospholipide in Frage.

Die in der Formulierungstechnik gebräuchlichen Tenside sind u.a. in folgenden Pulbikationen beschrieben:

«Mc Cutcheon's Detergents and Emulsifiers Annual» MC Publishing Corp., Ringwood, New Jersey, 1982.

Die pestiziden Zubereitungen enthalten in der Regel 0,01 bis 95%, insbesondere 0,1 bis 80%, Wirkstoff, 5 bis 99,99% eines festen oder flüssigen Zusatzstoffes und 0 bis 25%, insbesondere 0,1 bis 25%, eines Tensides.

Während als Handelsware eher konzentrierte Mitel bevorzugt werden, verwendet der Endverbraucher in der Regel verdünnte Mittel mit 1–10 000 ppm Wirkstoffgehalt.

Die Mittel können auch weitere Zusätze wie Stabilisatoren, Entschäumer, Viskositätsregulatoren, Bindemittel, Haftmittel sowie Dünger oder

andere Wirkstoffe zur Erzielung spezieller Effekte enthalten.

Die Verbindungen der Formel I stellen auch vielseitig reaktionsfähige Produkte zur Gewinnung weiterer Milbemycin-Derivate dar.

Beispiel 1

Herstellung aus Milbemycin D der Verbindungen $\Delta^{14,29}$-15-Hydroxi-milbemycin D (Formel Ia) und 14-Hydroxi-$\Delta$-$^{15,16}$-milbemycin D (Formel Ib)

Eine Lösung von 5,56 g Milbemycin D und 0,03 g Methylenblau in 400 ml Acetonitril wird in einer Bestrahlungsapparatur aus Glas unter Sauerstoffzugabe während 10 Stunden bei einer Temperatur von 20 °C (Projektorlampe 200 W) mit sichtbarem Licht bestrahlt.

Anschliessend wird im Reaktionsgemisch bei einer Temperatur von 20 °C mit 3,9 g Triphenylphosphin reduziert.

Das Reaktionsgemisch wird nach dem Einengen über einer Silicagelkolonne mit Methylenchlorid/Essigester = 3 : 1 als Eluiermittel chromatographiert.

Man erhält 4,10 g $\Delta^{14,29}$-15-Hydroxi-Milbemycin D, Smp. 228–229 °C; Massenspektrum m/e: 572 ($M^+$), 554.

Daneben erhält man 0,34 g 14-Hydroxi-$\Delta^{15,16}$-Milbemycin D, Smp. 252–254 °C; Massenspektrum m/e: 572 ($M^+$), 554.

Beispiel 2

Herstellung aus 5-Keto-milbemycin D der Verbindungen 5-Keto-$\Delta^{14,29}$-15-hydroxy-milbemycin D (Formel IA) und 5-Keto-14-hydroxi-$\Delta^{15,16}$-milbemycin D (Formel Ib)

a) Herstellung von 5-Keto-Milbemycin D

1 g Milbemycin D, 2 g aktiviertes Mangandioxid und 50 ml wasserfreies Methylenchlorid werden bei 20–25 °C 4 Stunden gerührt. Die Reaktionsmischung wird filtriert und das Filtrat wird über eine kurze (ca. 30 cm) Schicht Silicagel gereinigt. Man erhält 1 g 5-Keto-Milbemycin in Form einer gelblichen amorphen Masse, Smp. 152–157 °C.

b) Die Singulett-Oxydation des unter a) hergestellten 5-Keto-Milbemycins und die weitere Aufarbeitung erfolgt nach der im Beispiel 1 angegebenen Methode.

Man erhält nach Chromatographie an Silicagel 0,6 g 5-Keto-$\Delta^{14,29}$-15-hydroxi-milbemycin D, Smp. 160–165 °C; Massenspektrum m/e: 570 ($M^+$), 552.

Daneben erhält man 30 mg 5-Keto-14-hydroxi-$\Delta^{15,16}$-milbemycin D, Smp. 170–174 °C.

Beispiel 3

Herstellung aus Milbemycin D von 5-Keto-$\Delta^{14,29}$-15-hydroxi-milbemycin D (Formel Ia) und 5-Keto-14-hydroxi-$\Delta^{15,16}$-milbemycin D (Formel Ib)

Die Mangandioxid-Oxidation als Folgeschritt mit den nach Beispiel 1 durch Singulett-Sauerstoff-Oxidation gewonnenen Verbindungen $\Delta^{14,29}$-15-Hydroxi-milbemycin D und 14-Hydroxi-$\Delta^{15,16}$-milbemycin D liefert in quantitativer Ausbeute 5-Keto-$\Delta^{14,29}$-15-hydroxi-milbemycin D und 5-Keto-14-hydroxi-$\Delta^{15,16}$-milbemycin D.

Beispiel 4

Herstellung aus Milbemycin D von 5-Acetyl-$\Delta^{14,29}$-15-hydroxi-milbemycin D und von 5-Acetyl-14-hydroxy-$\Delta^{15,16}$-milbemycin D

a) Herstellung von 5-Acetyl-milbemycin D

560 g (1,0 mMol) Milbemycin D werden in 20 ml Pyridin mit 160 mg (1,6 mMol) Acetanhydrid versetzt und bei Raumtemperatur über Nacht gerührt. Nach dem Eindampfen des Pyridins wird der Rückstand in 20 ml Ethylacetat aufgenommen und die organische Phase einmal mit 10 ml in Chlorwasserstoffsäure-Lösung dann mit 10 ml gesättigter $NaHCO_3$-Lösung und zum Schluss mit 10 ml konz. NaCl-Lösung geschüttelt. Nach dem Abtrennen der organischen Phase wird diese über $Na_2SO_4$ getrocknet, filtriert und eingeengt. Man erhält 580 mg 5-Acetyl-milbemycin D (amorphes, schwachgelbes Pulver), Smp. 115–120 °C.

Auf analoge Art lassen sich auch die Acyl-Derivate Milbemycine $A_3$, $A_4$ und das 13-Desoxi-avermectin-Derivat ($R_2$ = sek.Butyl) gewinnen.

b) 560 mg 5-Acetyl-milbemycin D werden mit 20 mg Methylenblau in 40 ml Acetonitril in einer Bestrahlungsapparatur (Projektlampe 200 W) während 8 Stunden bei 18–22 °C mit Sauerstoff behandelt. Das Reaktionsgemisch wird anschliessend bei Raumtemperatur mit 40 mg Triphenylphosphin behandelt.

Nach dem Einengen wird das Reaktionsgemisch über einer Silicasäule (Laufmittel Methylenchlorid/Ethylacetat 3 : 1) chromatographiert.

Man erhält 390 mg 5-Acetyl-$\Delta^{14,29}$-15-hydroximilbemycin D, Smp. 153–156 °C; Massenspektrum m/e: 614 ($M^+$), 596.

Daneben erhält man 42 mg 5-Acetyl-14-hydroxi-$\Delta^{15,16}$-milbemycin D Smp. 151–154 °C.

Beispiel 5

Herstellung aus Milbemycin $A_4$ von $\Delta^{14,29}$-15-Hydroxi-milbemycin $A_4$ und 14-Hydroxi-$\Delta^{15,16}$-milbemycin $A_4$

540 mg (1 mMol) Milbemycin $A_4$ werden in 100 ml Acetonitril analog Beispiel 1 mit Singulett-Sauerstoff oxidiert und anschliessend mit Triphenylphosphin reduziert. Nach Reinigung durch Flash-Chromatographie an Silicagel (Laufmittel Cyclohexan/Ethylacetat 1 : 1) erhält man 310 mg $\Delta^{14,29}$-15-Hydroxi-milbemycin $A_4$, Smp. 222–225 °C; Massenspektrum m/e: 558 ($M^+$), 540.

Daneben erhält man 40 mg 14-Hydroxi-$\Delta^{15,16}$-milbemycin $A_4$, Smp. 147–152 °C; Massenspektrum m/e: 558 ($M^+$), 540.

Beispiel 6

Herstellung aus Milbemycin $A_3$ von 5-Dimethyl-tert.butyl-silyl-$\Delta^{14,29}$-15-hydroxi-milbemycin $A_3$ und von 5-Dimethyl-tert.butyl-silyl-14-hydroxi-$\Delta^{14,15}$-milbemycin $A_3$

a) Herstellung von 5-Dimethyl-tert.butylsilyl-milbemycin $A_3$.

Bei Raumtemperatur werden 480 mg (7 mMol) Imidazol und 460 mg (3 mMol) Dimethyl-tert.butylchlorsilan in 20 ml Methylenchlorid vorgelegt. Unter Rühren tropft man langsam eine Lösung von 655 mg (1,2 mMol) Milbemycin $A_3$ in 10 ml Methy-

lenchlorid dazu. Anschliessend wird das Reaktionsgemisch über Nacht unter Rückfluss (~40 °C) erwärmt. Nach dem Einengen, Reinigen über Silicagel und Trocknen erhält man 730 mg amorphes 5-Dimethyl-tert.butylsilyl-milbemycin $A_3$, Smp. 55–60 °C.

Auf gleiche Weise lassen sich auch die Milbemycine $A_4$, D und das 13-Desoxi-Avermectin-Derivat ($R_2$ = sek.Butyl) silylieren, wobei auch vorteilhaft Methyldiphenylchlorsilan oder bis(Isopropyl)-methylchlorsilan eingesetzt werden können.

b) Nach der Vorschrift des Beispiels 4b) werden aus 720 mg 5-Dimethyl-tert.butylsilyl-milbemycin $A_3$ durch Singulett-Sauerstoff-Oxidation mit Bengalrosa als Sensibilisator und anschliessende Reduktion der Peroxide mit Triphenylphosphin 550 mg 5-Dimethyl-tert.butylsilyl-$\Delta^{14,29}$-15-hydroxi-milbemycin $A_3$, gewonnen, Smp. 238–240 °C; Massenspektrum m/e: 658 ($M^+$), 640.

Daneben werden 42 mg 5-Dimethyl-tert.butylsilyl-14-hydroxi-$\Delta^{15,16}$-milbemycin $A_3$ in amorpher Form erhalten, Smp. 45–50 °C.

Beispiel 6

Herstellung von $\Delta^{14,29}$-15-Hydroxi-milbemycin $A_3$ und von 14-Hydroxi-$\Delta^{15,16}$-milbemycin $A_3$

120 mg 5-Dimethyl-tert.butylsilyl-$\Delta^{14,29}$-15-hydroxi-milbemycin $A_3$ werden mit 2 ml einer 1%igen Lösung von p-Toluolsulfonsäure in Methanol 9 Stunden bei Raumtemperatur gerührt und dann mit 5%iger wässriger $NaHCO_3$-Lösung behandelt. Durch Ausschütteln mit dreimal 2 ml Diethylether, Einengen der organischen Phase und Chromatographie des Rohprodukts an 20 g Kieselgel (Laufmittel Aceton/Methylenchlorid 1 : 12) erhält man 67 mg $\Delta^{14,29}$-15-Hydroxi-milbemycin $A_3$, Smp. 219–222 °C.

Entsprechend erhält man aus 60 mg 5-Dimethyl-tert. butylsilyl-14-hydroxi-$\Delta^{15,16}$-milbemycin $A_3$ 38 mg 14-Hydroxi-$\Delta^{15,16}$-milbemycin $A_3$, Smp. 128–132 °C.

Nach Art der vorhergehenden Beispiele lassen sich Verbindungen der Formel I herstellen. In der folgenden Tabelle bedeuten

Ia = $\Delta^{14,29}$-15-ol-Derivat

Ib = 14-Hydroxi-$\Delta^{15,16}$-Derivat;

Sofern in der Spalte für $R_1$ eine Angabe fehlt, handelt es sich um ein 5-Keto-milbemycin (X = CO). $^1$H-NMR-Daten werden in $CDCl_3$ bei 250 MHZ ermittelt, wobei $Si(CH_3)_4$ als Referenz-Substanz diente.

| Verb. Nr. | A | $R_1$ | $R_2$ | Physikalische Daten |
|---|---|---|---|---|
| 1.1 | Ia | $-OH$ | $isoC_3H_7$ | Smp. 228–229°C |
| 1.2 | Ib | $-OH$ | " | Smp. 252–254°C |
| 1.3 | Ia | $-OSi(CH_3)_2t.C_4H_9$ | " | Smp. 235–238°C |
| 1.4 | Ib | " | " | Smp. 145–150°C |
| 1.5 | Ia | $-OSiCH_3(C_6H_5)_2$ | " | |

| Verb. Nr. | A | $R_1$ | $R_2$ | Physikalische Daten |
|---|---|---|---|---|
| 1.6 | Ib | $-OSiCH_3(C_6H_5)_2$ | $isoC_3H_7$ | |
| 1.7 | Ia | $-O-COCH_3$ | " | Smp. 153–156°C |
| 1.8 | Ib | $-O-COCH_3$ | " | Smp. 151–154°C |
| 1.9 | Ia | $-O-COC_2H_5$ | " | Smp. 157–160°C |
| 1.10 | Ib | $-O-COC_2H_5$ | " | Smp. 155–159°C |
| 1.11 | Ia | – | " | Smp. 160–165°C |
| 1.12 | Ib | – | " | Smp. 170–174°C |
| 1.13 | Ia | $-OSi(CH_3)_3$ | " | |
| 1.14 | Ib | $-OSi(CH_3)_3$ | " | |

| Verb. Nr. | A | $R_1$ | $R_2$ | Physikalische Daten |
|---|---|---|---|---|
| 2.1 | Ia | $-OH$ | $C_2H_5$ | Smp. 222–225°C |
| 2.2 | Ib | $-OH$ | " | Smp. 147–152°C |
| 2.3 | Ia | $-OSi(CH_3)_2t.C_4H_9$ | " | amorph |
| 2.4 | Ib | " | " | Smp. 58–62°C |
| 2.5 | Ia | $-O-SO_2CH_3$ | " | |
| 2.6 | Ib | $-O-SO_2CH_3$ | " | |
| 2.7 | Ia | $-O-COCH_3$ | " | Smp. 158–161°C |

| Verb. Nr. | A | $R_1$ | $R_2$ | Physikalische Daten |
|---|---|---|---|---|
| 2.8 | Ib | $-O-COCH_3$ | $C_2H_5$ | Smp. 156–160°C |
| 2.9 | Ia | $-O-COC(CH_3)_3$" | | |
| 2.10 | Ib | $-O-COC(CH_3)_3$ | " | |
| 2.11 | Ia | – | " | NMR:4,91(s);5,24(s) ($C_{13}=CH_2$) |
| 2.12 | Ib | – | " | amorph |

| Verb. Nr. | A | $R_1$ | $R_2$ | Physikalische Daten |
|---|---|---|---|---|
| 3.1 | Ia | –OH | $CH_3$ | Smp. 220–223°C |
| 3.2 | Ib | –OH | $CH_3$ | amorph |
| 3.3 | Ia | $-OSi(CH_3)_2t.C_4H_9$ | $CH_3$ | Smp. 238–240°C |
| 3.4 | Ib | " | $CH_3$ | Smp. 45-50°C |
| 3.5 | Ia | $-O-COCH_3$ | $CH_3$ | |
| 3.6 | Ib | $-O-COCH_3$ | $CH_3$ | |
| 3.7 | Ia | $-O-COC_6H_5$ | $CH_3$ | |
| 3.8 | Ib | $-O-COC_6H_5$ | $CH_3$ | |
| 3.9 | Ia | $-O-SO_2C_6H_5$ | $CH_3$ | amorph |
| 3.10 | Ib | $-O-SO_2C_6H_5$ | $CH_3$ | |

| Verb. Nr. | A | $R_1$ | $R_2$ | Physikalische Daten |
|---|---|---|---|---|
| 3.11 | Ia | – | $CH_3$ | Smp. 145–148°C |
| 3.12 | Ib | – | $CH_3$ | Smp. 152–156°C |

| Verb. Nr. | A | $R_1$ | $R_2$ | Physikalische Daten |
|---|---|---|---|---|
| 4.1 | Ia | –OH | sek.$C_4H_9$ | NMR:4,92(s);5,25(s) ($C_{13}=CH_2$) |
| 4.2 | Ib | –OH | sek.$C_4H_9$ | |
| 4.3 | Ia | – | sek.$C_4H_9$ | NMR:4,91(s);5,25(s) ($C_{13}=CH_2$) |
| 4.4 | Ib | – | sek.$C_4H_9$ | |

Formulierungsbeispiele für Wirkstoffe der Formel I
(% = Gewichtsprozent)

| Spritzpulver | a) | b) | c) |
|---|---|---|---|
| Wirkstoff gemäss vorstehender Tabelle | 25% | 50% | 75% |
| Na-Ligninsulfonat | 5% | 5% | – |
| Na-Laurylsulfat | 3% | – | 5% |
| Na-Diisobutylnapthalinsulfonat | – | 6% | 10% |
| Octylphenolpolyäthylenglykoläther (7-8 Mol AeO) | – | 2% | – |
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff gemäss Tabelle | 10% |
| Octylphenolpolyäthylenglykoläther (4–5 Mol AeO) | 3% |
| Ca-Dodecylbenzolsulfonat | 3% |

| | |
|---|---|
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| Stäubemittel | a) | b) |
|---|---|---|
| Wirkstoff gemäss Tabelle | 5% | 8% |
| Talkum | 95% | – |
| Kaolin | – | 92% |

Man erhält anwendungsfertige Stäubemittel, indem der Wirkstoff mit dem Träger vermischt und auf einer geeigneten Mühle vermahlen wird.

| Extruder Granulat | |
|---|---|
| Wirkstoff gemäss Tabelle | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| Umhüllungs-Granulat | |
|---|---|
| Wirkstoff gemäss Tabelle | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| Suspensions-Konzentrat | |
|---|---|
| Wirkstoff gemäss Tabelle | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Falls die Verbindungen der Formel I bzw. entsprechende Mittel zur Bekämpfung von endoparasitären Nematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Die Wirkstoffe bzw. die sie enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew.-%. Die Mittel können in Form von Lösungen, Emulsionen, Suspensionen, Pulver, Tabletten, Bolussen oder Kapseln den Tieren peroral oder intraruminal verabreicht werden.

Soweit die physikalischen und toxikologischen Eigenschaften von Lösungen oder Emulsionen dies zulassen, können die Verbindungen der Formel I bzw. sie enthaltende Mittel an Tieren auch beispielsweise subcutan injiziert oder mittels der Pour-on-Methode auf den Körper der Tiere appliziert werden. Ferner ist eine Verabreichung der Wirkstoffe an die Tiere auch mittels Lecksteinen (Salz) oder Molasse-Blöcken möglich.

Biologische Beispiele

B-1. Insektizide Frassgift-Wirkung bei Spodoptera littoralis

Baumwollpflanzen werden mit einer Versuchslösung besprüht, die 3; 12,5 oder 50 ppm der zu prüfenden Verbindung enthält.

Nach dem Antrocknen des Belags werden die Pflanzen mit Larven ($L_1$-Stadium) von Spodoptera littoralis besetzt. Pro Versuchsverbindung und pro Test-Spezies verwendet man zwei Pflanzen. Der Versuch wird bei ca. 24 °C und 60% relativer Luftfeuchtigkeit durchgeführt. Auswertungen und Zwischenauswertungen erfolgen nach 24 Std., 48 Std. und 72 Stunden.

Die Verbindungen Nr. 1.1, 1.2, 1.7, 1.11, 1.12, 1.13, 2.1, 2.2, 2.5, 2.7, 2.11, 2.12, 3.1, 3.2, 3.11, 4.1 und 4.3 erzielen bei Wirkstoffkonzentrationen von 12,5 ppm eine vollständige Abtötung nach 24 Stunden.

B-2. Wirkung gegen pflanzenschädigende Akariden:

OP-sensible Tetranychus urticae

Die Primärblätter von Bohnenpflanzen (Phaseolus vulgaris) werden 16 Std. vor dem Versuch mit einem durch T. urticae infestierten, aus einer Massenzucht stammenden Blattstück belegt. Die so künstlich infestierten Pflanzen werden nach Entfernung des Blattstücks mit einer Versuchslösung bis zur Tropfnässe besprüht, die wahlweise 0,4 ppm oder 1,6 ppm der zu prüfenden Verbindung enthält. Die Temperatur in der Gewächshauskabine beträgt ca. 25 °C.

Nach einem und nach acht Tagen werden Imagines und Larven unter dem Binokular auf die Zahl lebender und toter Individuen untersucht.

Die Verbindungen Nr. 1.1, 1.2, 1.3, 1.4, 1.7, 1.8, 1.9, 1.11, 1.12, 2.1, 2.2, 2.3, 2.7, 2.11, 3.1, 3.2, 3.3, 3.4, 3.11, 3.12, 4.1 und 4.3 erzielen bei Wirkstoffkonzentrationen von 1,6 ppm nach 24 Stunden vollständige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50 °C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt.

Die Verbindungen Nr. 1.1, 1.2, 1.11, 1.12, 2.1, 2.2, 2.3, 2.7, 2.11, 3.1, 3.2, 4.1 und 4.3 erzielen mit 250 ppm eine Wirkung von 100%.

B-4. Akarizide Wirkung gegen Boophilus microplus (Biarra-Stamm)

Auf einer PVC-Platte wird waagerecht ein Klebestreifen so befestigt, dass darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1 : 1-Mischung von Polyethylenglykol und Aceton darstellt und in der eine bestimmte Wirkstoffmenge von wahlweise 1, 0.1 oder 0.01 µg pro Zecke gelöst ist. Kontrolltiere erhalten eine wirkstofffreie Injektion. Nach der Behandlung werden die Tiere von der Unterlage abgelöst und unter Normalbedingungen in einem Insektarium bei ca. 28 °C und 80% rel. Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90%) Eier ablegen, die nicht schlupffähig sind.

Die Verbindungen Nr. 1.1, 1.2, 1.3, 1.7, 1.9, 1.10, 1.11, 1.12, 2.1, 2.2, 2.3, 2.7, 2.11, 2.12, 3.1, 3.2, 3.3, 3.4, 3.9, 4.1, 4.2 und 4.3 erzielten eine $IR_{90}$ von 0.1 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trichostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 2 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen Nr. 1.1–1.4, 1.7–1.12, 2.1–2.4, 2.7, 2.8, 2.11, 2.12, 3.1–3.4, 3.11, 3.12, 4.1 und 4.3 bei 2 mg/kg behandelt worden sind, zeigen im Vergleich zu unbehandelten, aber infizierten Vergleichsgruppen einen um 90 bis 100% reduzierten Nematodenbefall.

**Patentansprüche für die Vertragsstaaten BE, CH, DE, FR, GB, IT, LI, NL, SE**

1. Milbemiycin-Derivate der Formel I

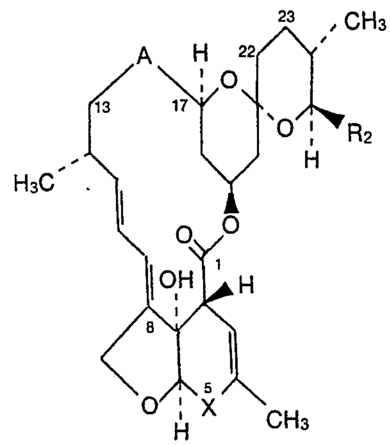

(I)

worin A die Struktur

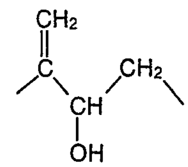

[$=\Delta^{14,29}$–15–ol]

oder die Struktur

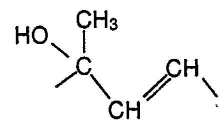

[= 14-Hydroxi-$\Delta^{15,16}$]

bedeutet,

während X entweder $-CH(OR_1)-$ oder $-CO-$ ist, und $R_1$ Wasserstoff, eine Silylgruppe der Formel

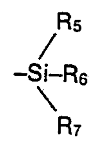

darstellt, worin $R_5$ einen $C_1$–$C_4$-aliphatischen Rest oder Benzyl und $R_6$ und $R_7$ unabhängig voneinander einen $C_1$–$C_4$-aliphatischen Rest, Benzyl oder Phenyl bedeuten oder für die Acylgruppen $R_3$–CO– oder $R_4$–$SO_2$– steht,

worin $R_3$ einen unsubstituierten oder halogenierten $C_1$–$C_6$-aliphatischen Rest oder unsubstituiertes oder durch $C_1$–$C_4$-Alkyl oder Halogen substituiertes Phenyl beudetet und $R_4$ einen $C_1$–$C_4$-Alkylrest oder einen unsubstituierten oder durch Methyl, Chlor oder Nitro substituierten Phenylrest darstellt;

$R_2$ Methyl, Ethyl, Isopropyl oder sek.Butyl darstellen.

2. Verbindungen der Formel I gemäss Anspruch 1, worin X entweder $-CH(OR_1)-$ oder $-CO-$ ist, $R_1$ Wasserstoff bedeutet und $R_2$ Isopropyl darstellt.

3. Verbindungen der Formel I gemäss Anspruch 1, worin A $\Delta^{14,29}$-15-ol bedeutet.

4. Verbindungen der Formel I gemäss Anspruch 3, worin X entweder –CHOH– oder –CO– bedeutet.

5. Verbindungen der Formel I gemäss Anspruch 3 worin $R_1$ die vorgenannte Silylgruppe darstellt, in der $R_5$ Methyl, Ethyl, Propyl, Isopropyl, tert.Butyl, und $R_6$ und $R_7$ unabhängig Methyl, Ethyl, Isopropyl, tert.Butyl, Phenyl oder Benzyl bedeuten, während $R_2$ Methyl, Ethyl, Isopropyl oder sek.Butyl ist.

6. Verfahren zur Herstellung von Verbindungen der Formel I gemäss Anspruch 1, dadurch gekennzeichnet, dass man eine Verbindung der Formel II

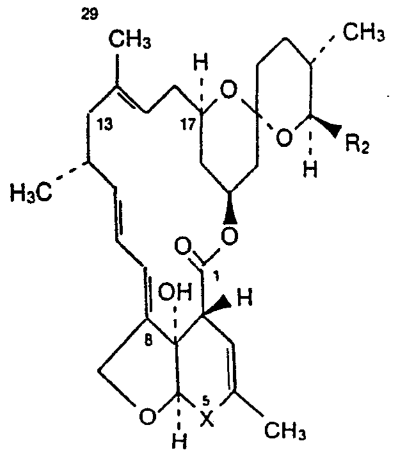

(II),

worin X und $R_2$ die für Formel I gegebene Bedeutung haben, durch Singulett-Sauerstoff oxidiert und die intermediär entstehenden 15-Peroxide und 14-Peroxide reduziert.

7. Verfahren gemäss Anspruch 6, wobei die Reaktion bei sichtbarem Licht in Gegenwart eines Sensibilisators bei Normaldruck im Temperaturbereich von $-90\,°C$ bis $+45\,°C$ in einem inerten Lösungsmittel durchgeführt wird.

8. Verfahren gemäss Anspruch 7, wobei die Reaktion im Temperaturbereich von $0\,°C$ bis $+20\,°C$ durchgeführt wird.

9. Verfahren gemäss Anspruch 6, wobei die Reaktion in einer Bestrahlungsapparatur mit einer Lampe von 60–500 Watt durchgeführt wird.

10. Verfahren gemäss Anspruch 6, wobei der Reduktionsschritt mit $NaBH_4$, $LiA1H_4$ oder $P(C_6H_5)_3$ durchgeführt wird.

11. Verfahren gemäss Anspruch 6 zur Herstellung von Milbemycin D-Derivaten der Formel I, worin A die genannten Bedeutungen hat, X entweder –CHOH– oder –CO– darstellt und $R_2$ Isopropyl ist, durch Singulett-Sauerstoff-Oxidation von Milbemycin D oder 5-Keto-milbemycin D und anschliessende Reduktion.

12. Schädlingsbekämpfungsmittel, welches als mindestens eine aktive Komponente eine Verbindung gemäss Anspruch 1 zusammen mit geeigneten Trägerstoffen und/oder Verteilungsmitteln enthält.

13. Mittel gemäss Anspruch 12, welches als mindestens eine aktive Komponente eine Verbindung gemäss einem der Ansprüche 2 bis 5 enthält.

14. Eine Verbindung der Formel I gemäss einem der Ansprüche 1 bis 5 zur Bekämpfung von Ekto- und Endoparasiten an Pflanzen und Tieren.

15. Eine Verbindung der Formel I gemäss Anspruch 14 zur Bekämpfung von Endoparasiten im Warmblüter.

16. Eine Verbindung der Formel I gemäss Anspruch 15, zur Bekämpfung von Nematoden.

17. Eine Verbindung der Formel I gemäss einem der Ansprüche 1 bis 5 zur Bekämpfung von Ektoparasiten an Tieren oder Pflanzenparasiten, die man in Aufwandmengen von 10 bis 1000 g per Hektar auf geschlossene Kulturanbauflächen, in Pferchen, Stallungen oder sonstigen Räumen appliziert.

**Patentansprüche für den Vertragsstaat AT**

1. Schädlingsbekämpfungsmittel enthaltend neben geeigneten Trägermaterialien als aktive Komponente mindestens ein Milbemycin-Derivat der Formel I

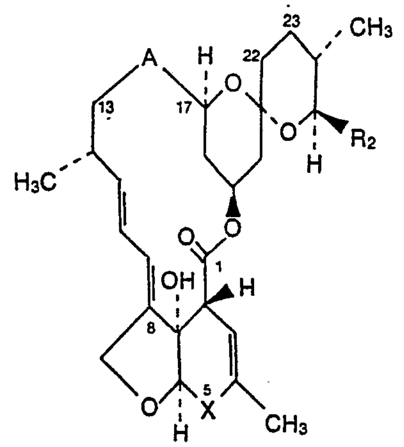

(I)

worin A die Struktur

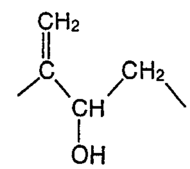

$[=\Delta^{14.29}$–15–ol]

oder die Struktur

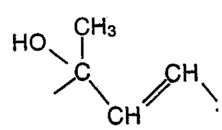

[= 14-Hydroxi–$\Delta^{15,\,16}$]

bedeutet,

worin X entweder –CH($OR_1$)– oder –CO– ist, und $R_1$ Wasserstoff, eine Silylgruppe der Formel

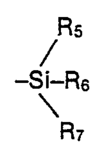

darstellt, worin $R_5$ einen $C_1$–$C_4$-aliphatischen Rest oder Benzyl und $R_6$ und $R_7$ unabhängig voneinander einen $C_1$–$C_4$-aliphatischen Rest, Benzyl oder Phenyl bedeuten oder für die Acylgruppen $R_3$–CO– oder $R_4$–$SO_2$– steht,

worin $R_3$ einen unsubstituierten oder halogenierten $C_1$–$C_6$-aliphatischen Rest oder unsubstituiertes oder durch $C_1$–$C_4$-Alkyl oder Halogen substituiertes Phenyl bedeutet und $R_4$ einen $C_1$–$C_4$-Alkylrest oder einen unsubstituierten oder durch Methyl, Chlor oder Nitro substituierten Phenylrest darstellt;

$R_2$ Methyl, Ethyl, Isopropyl oder sek.Butyl darstellen.

2. Mittel nach Anspruch 1, enthaltend als aktive Komponente eine Verbindung der Formel I, worin X entweder –CH($OR_1$)– oder –CO– ist, $R_1$ Wasserstoff bedeutet und $R_2$ Isopropyl darstellt.

3. Mittel nach Anspruch 1, enthaltend als aktive Komponente eine Verbindung der Formel I, worin A $\Delta^{14,29}$-15-ol bedeutet.

4. Mittel nach Anspruch 3, enthaltend als aktive Komponente eine Verbindung der Formel I, worin X entweder –CHOH– oder –CO– bedeutet.

5. Mittel nach Anspruch 3, enthaltend als aktive Komponente einer Verbindung der Formel I, worin $R_1$ die vorgenannte Silylgruppe darstellt, in der $R_5$ Methyl, Ethyl, Propyl, Isopropyl, tert.Butyl, und $R_6$ und $R_7$ unabhängig Methyl, Ethyl, Isopropyl, tert.-Butyl, Phenyl oder Benzyl bedeuten, während $R_2$ Methyl, Ethyl, Isopropyl oder sek.Butyl ist.

6. Verfahren zur Herstellung von Verbindungen der Formel I gemäss Anspruch 1, dadurch gekennzeichnet, dass man eine Verbindung der Formel II

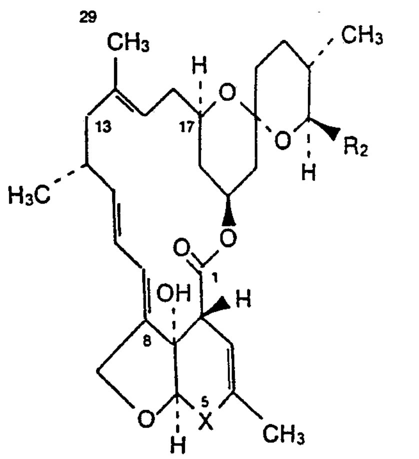 (II),

worin X und $R_2$ die für Formel I gegebene Bedeutung haben, durch Singulett-Sauerstoff oxidiert und die intermediär entstehenden 15-Peroxide und 14-Peroxide reduziert.

7. Verfahren gemäss Anspruch 6, wobei die Reaktion bei sichtbarem Licht in Gegenwart eines Sensibilisators bei Normaldruck im Temperaturbereich von $-90\,°C$ bis $+45\,°C$ in einem inerten Lösungsmittel durchgeführt wird.

8. Verfahren gemäss Anspruch 7, wobei die Reaktion im Temperaturbereich von $0\,°C$ bis $+20\,°C$ durchgeführt wird.

9. Verfahren gemäss Anspruch 6, wobei die Reaktion in einer Bestrahlungsapparatur mit einer Lampe von 60–500 Watt durchgeführt wird.

10. Verfahren gemäss Anspruch 6, wobei der Reduktionsschritt mit $NaBH_4$, $LiAlH_4$ oder $P(C_6H_5)_3$ durchgeführt wird.

11. Verfahren gemäss Anspruch 6 zur Herstellung von Milbemycin D-Derivaten der Formel I, worin A die genannten Bedeutungen hat, X entweder –CHOH– oder –CO– darstellt und $R_2$ Isopropyl ist, durch Singulett-Sauerstoff-Oxidation von Milbemycin D oder 5-Keto-milbemycin D und anschliessende Reduktion.

12. Mittel gemäss einem der Ansprüche 1 bis 5 zur Bekämpfung von Ekto- und Endoparasiten an Pflanzen und Tieren.

13. Mittel gemäss Anspruch 12 zur Bekämpfung von Endoparasiten im Warmblüter.

14. Mittel gemäss Anspruch 13, zur Bekämpfung von Nematoden.

15. Ein Mittel gemäss einem der Ansprüche 1 bis 5 zur Bekämpfung von Ektoparasiten an Tieren oder von Pflanzenparasiten, das man in Aufwandmengen in Bezug auf den Wirkstoff von 10 bis 1000 g per Hektar auf geschlossene Kulturanbauflächen, in Pferchen, Stallungen oder sonstige Räume appliziert.

**Patent Claims for the contracting states BE, CH, DE, FR, GB, IT, LI, NL, SE.**

1. A milbemycin derivative of the formula I

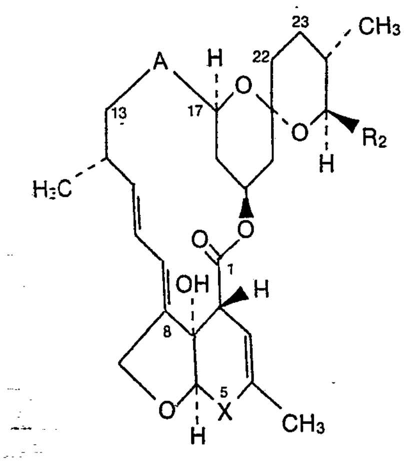 (I)

wherein A has the structure

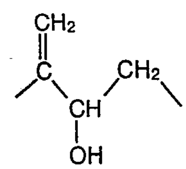 [$=\Delta^{14,29}$–15–ol]

or the structure

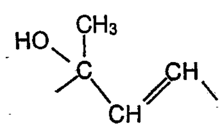 [=14-Hydroxy–$\Delta^{15,16}$]

whilst X is either –CH($OR_1$)– or –CO–, and

$R_1$ is hydrogen, a silyl group of the formula

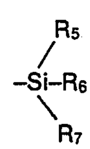

in which $R_5$ is a $C_1$–$C_4$-aliphatic radical or benzyl and $R_6$ and $R_7$ are each independently of the other a $C_1$–$C_4$-aliphatic radical, benzyl or phenyl, or $R_1$ represents the acyl group $R_3$–CO– or $R_4$–$SO_2$– in which $R_3$ is an unsubstituted or halogenated $C_1$–$C_6$-aliphatic radical or phenyl that is unsubstituted or is substituted by $C_1$–$C_4$-alkyl or by halogen and $R_4$ is a $C_1$–$C_4$-alkyl radical or a phenyl radical that is unsubstituted or is substituted by methyl, chlorine or by nitro; and

$R_2$ is methyl, ethyl, isopropyl or sec.butyl.

2. Compounds of the formula I according to claim 1, wherein X is either –CH($OR_1$)– or –CO–, $R_1$ is hydrogen and $R_2$ is isopropyl.

3. Compounds of the formula I according to claim 1, wherein A is $\Delta^{14,29}$-15-ol.

4. Compounds of the formula I according to claim 3, wherein X is either –CHOH– or –CO–.

5. Compounds of the formula I according to claim 3, wherein $R_1$ is the above-mentioned silyl group in which $R_5$ is methyl, ethyl, propyl, isopropyl or tert-butyl, and $R_6$ and $R_7$ are each independently of the other methyl, ethyl, isopropyl, tert-butyl, phenyl or benzyl, whilst $R_2$ is methyl, ethyl, isopropyl or sec-butyl.

6. A process for the preparation of compounds of the formula I according to claim 1, which comprises oxidising a compound of the formula II

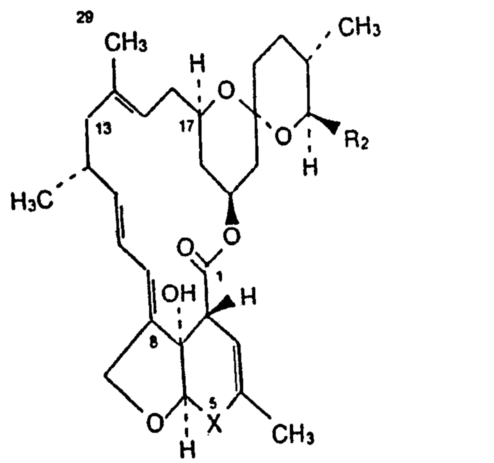

(II)

wherein X and $R_2$ are as defined for formula I, by singlet oxygen oxidation, and reducing the 15-peroxide and 14-peroxide obtained as intermediates.

7. A process according to claim 6, wherein the reaction is carried out in visible light, in the presence of a sensitiser, under normal pressure and in the temperature range from −90 °C to +45 °C, in an inert solvent.

8. A process according to claim 7, wherein the reaction is carried out in the temperature range from 0 °C to +20 °C.

9. A process according to claim 6, wherein the reaction is carried out in an irradiation apparatus with a 60–500 watt lamp.

10. A process according to claim 6, wherein the reduction step is carried out with $NaBH_4$, $LiAlH_4$ or $P(C_6H_5)_3$.

11. A process according to claim 6 for the preparation of milbemycin D derivatives of the formula I, wherein A has the meanings given, X is either –CHOH– or –CO– and $R_2$ is isopropyl, by singlet oxygen oxidation of milbemycin D or 5-keto-milbemycin D and subsequent reduction.

12. A pesticidal composition which contains as at least one active ingredient a compound according to claim 1, together with suitable carriers and/or diluents.

13. A composition according to claim 12 which contains as at least one active ingredient a compound according to any one of claims 2 to 5.

14. A compound of formula I according to any one of claims 1 to 5 for controlling ecto- and endoparasites of plants and animals.

15. A compound of formula I according to claim 14 for controlling endoparasites in warm-blooded animals.

16. A compound of formula I according to claim 15 for controlling nematodes.

17. A compound of formula I according to any one of claims 1 to 5 for controlling ectoparasites of animals or plant parasites, which is applied at application rates of from 10 to 1000 g per hectare to enclosed crop areas, pens, livestock buildings or other buildings.

**Claims for the contracting state AT**

1. A pesticidal composition which, in addition to suitable carriers, contains as active ingredient at least one milbemycin derivative of the formula I

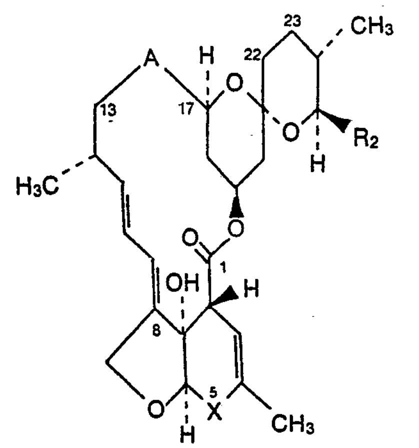

(I)

wherein A has the structure

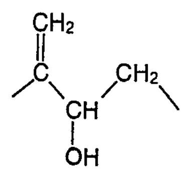

[$=\Delta^{14,29}$–15–ol]

or the structure

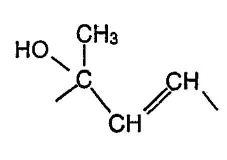

[=14-Hydroxy-$\Delta^{15,16}$]

whilst X is either –CH($OR_1$)– or –CO–, and

$R_1$ is hydrogen, a silyl group of the formula

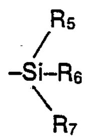

in which $R_5$ is a $C_1$–$C_4$-aliphatic radical or benzyl and $R_6$ and $R_7$ are each independently of the other a $C_1$–$C_4$-aliphatic radical, benzyl or phenyl, or $R_1$ represents the acyl group $R_3$–CO– or $R_4$–$SO_2$– in which $R_3$ is an unsubstituted or halogenated $C_1$–$C_6$-aliphatic radical or phenyl that is unsubstituted or is substituted by $C_1$–$C_4$-alkyl or by halogen and $R_4$ is a $C_1$–$C_4$-alkyl radical or a phenyl radical that is unsubstituted or is substituted by methyl, chlorine or by nitro; and

$R_2$ is methyl, ethyl, isopropyl or sec.butyl.

2. A composition according to claim 1 containing as active ingredient a compound of the formula I wherein X is either $-CH(OR_1)-$ or –CO–, $R_1$ is hydrogen and $R_2$ is isopropyl.

3. A composition according to claim 1 containing as active ingredient a compound of the formula I wherein A is $\Delta^{14,29}$-15-ol.

4. A composition according to claim 3 containing as active ingredient a compound of the formula I wherein X is either –CHOH– or –CO–.

5. A composition according to claim 3 containing as active ingredient a compound of the formula I wherein $R_1$ is the above-mentioned silyl group in which $R_5$ is methyl, ethyl, propyl, isopropyl or tert-butyl, and $R_6$ and $R_7$ are each independently of the other methyl, ethyl, isopropyl, tert-butyl, phenyl or benzyl, whilst $R_2$ is methyl, ethyl, isopropyl or sec-butyl.

6. A process for the preparation of compounds of the formula I according to claim 1, which comprises oxidising a commpound of the formula II

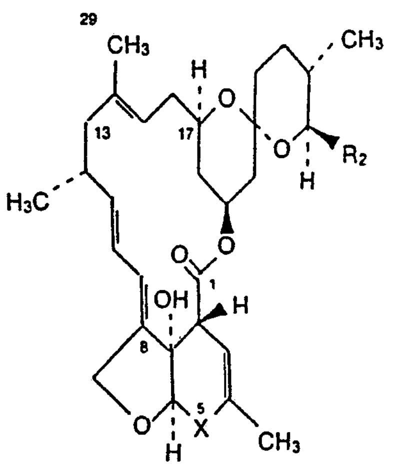

(II)

wherein X and $R_2$ are as defined for formula I, by singlet oxygen oxidation, and reducing the 15-peroxide and 14-peroxide obtained as intermediates.

7. A process according to claim 6, wherein the reaction is carried out in visible light, in the presence of a sensitiser, under normal pressure and in the temperature range from −90 °C to +45 °C, in an inert solvent.

8. A process according to claim 7, wherein the reaction is carried out in the temperature range from 0 °C to +20 °C.

9. A process according to claim 6, wherein the reaction is carried out in an irradiation apparatus with a 60–500 watt lamp.

10. A process according to claim 6, wherein the reduction step is carried out with $NaBH_4$, $LiAlH_4$ or $P(C_6H_5)_3$.

11. A process according to claim 6 for the preparation of milbemycin D derivatives of the formula I, wherein A has the meanings given, X is either –CHOH– or –CO– and $R_2$ is isopropyl, by singlet oxygen oxidation of milbemycin D or 5-keto-milbemycin D and subsequent reduction.

12. A composition according to any one of claims 1 to 5 for controlling ecto- and endo-parasites of plants and animals.

13. A composition according to claim 12 for controlling endoparasites in warm-blooded animals.

14. A composition according to claim 13 for controlling nematodes.

15. A composition according to any one of claims 1 to 5 for controlling ectoparasites of animals or plant parasites, which is applied at application rates relating to the active ingredient of from 10 to 1000 g per hectare to enclosed crop areas, pens, livestock buildings or other buildings.

**Revendications pour les Etats Contractants BE, CH, DE, FR, GB, IT, LI, NL, SE**

1. Dérivé de milbémycine de formule I

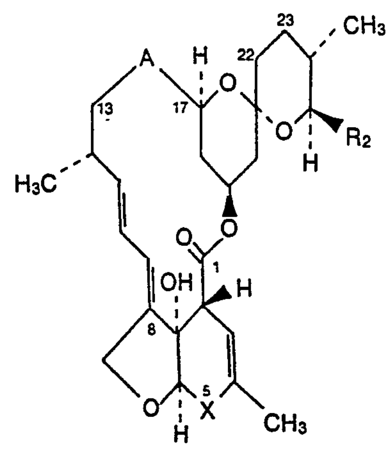

(I)

dans laquelle A est la structure

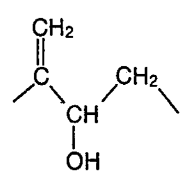

[$=\Delta^{14,29}$–15–ol]

ou la structure

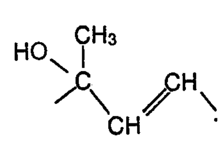

[=14-Hydrox –$\Delta^{15,16}$]

tandis que X est soit 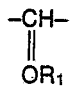 soit 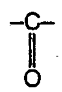

et $R_1$ est l'hydrogène, un groupe silyle de formule

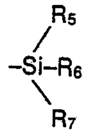

dans lequel $R_5$ est un reste aliphatique $C_1$–$C_4$ ou un benzyle et $R_6$ et $R_7$ représentent indépendamment l'un de l'autre un reste aliphatique $C_1$–$C_4$, benzyle ou phényle ou représente les groupes acyles $R_3$–CO– ou $R_4$–$SO_2$– dans laquelle $R_3$ est un reste aliphatique $C_1$–$C_6$ non substitué ou halogéné ou un phényle non substitué par un alkyle $C_1$–$C_4$ ou un halogène et $R_4$ est un reste alkyle $C_1$–$C_4$ ou un reste phényle non substitué ou substitué par le méthyle, chlore ou nitro;

$R_2$ représente le méthyle, l'éthyle, l'isopropyle ou le sec. butyle.

2. Composés de formule I selon la revendication 1 dans laquelle X est soit –CH($OR_1$)– soit –CO–, $R_1$ représente l'hydrogène et $R_2$ représente l'isopropyle.

3. Composés de formule I selon la revendication 1 dans laquelle A représente $\Delta^{14,29}$-15-OL.

4. Composés de formule I selon la revendication 3 dans laquelle X représente soit –CHOH– soit –CO–.

5. Composés de formule I selon la revendication 3 dans laquelle $R_1$ représente le groupe silyle cité ci-dessus, dans lequel $R_5$ est le méthyle, l'éthyle, le propyle, l'isopropyle, le tert. butyle, et $R_6$ et $R_7$ représentent indépendamment le méthyle, l'éthyle, l'isopropyle, le tert. butyle, le phényle ou le benzyle, tandis que $R_2$ est le méthyle, l'éthyle, l'isopropyle ou le sec. butyle.

6. Procédé de préparation de composés de formule I selon la revendication 1, caractérisé en ce qu'on oxyde un composé de formule II

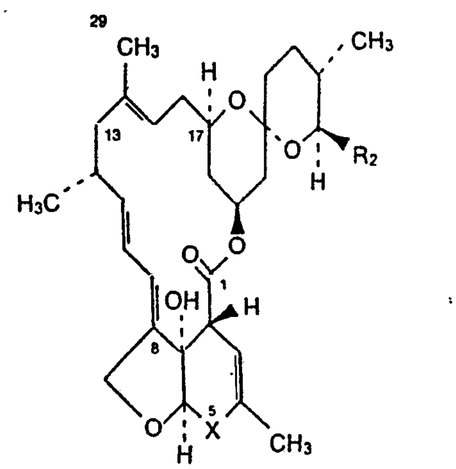

(II),

dans laquelle X et $R_2$ ont la signification donnée pour la formule I, par de l'oxygène radicalaire et qu'on réduit les 15-peroxydes et 14-peroxydes.

7. Procédé selon la revendication 6, dans lequel la réaction est réalisée par la lumière visible en présence d'un sensibilisateur à pression normale dans l'intervalle de température de −90 °C à +45 °C dans un solvant inerte.

8. Procédé selon la revendication 7, dans lequel la réaction est réalisée dans l'intervalle de température de 0 °C à +20 °C.

9. Procédé selon la revendication 6, dans lequel la réaction est réalisée dans un appareil à irradiation avec une lampe de 60–500 watt.

10. Procédé selon la revendication 6, dans lequel l'étape de réduction est réalisée avec $NaBH_4$, $LiAlH_4$ ou $P(C_6H_5)_3$.

11. Procédé selon la revendication 6, pour préparer des dérivés de milbémycine D de formule I, dans lesquels A a les significations indiquées, X représente soit –CHOH– soit –CO– et $R_2$ est l'isopropyle, par oxydation par l'oxygène radicalaire de milbémycine D ou de 5-céto-milbémycine D et réduction successive.

12. Moyen de lutte anti-parasitaire qui contient au moins comme composant actif un composé selon la revendication 1 avec les véhicules et/ou moyens de dispersion appropriés.

13. Moyen selon la revendication 12 qui contient au moins comme composant actif un composé selon l'une des revendications 2 à 5.

14. Composé de formule I selon l'une des revendications 1 à 5 pour combattre des ecto- et endoparasites de plantes et d'animaux.

15. Composé de formule I selon la revendication 14 pour combattre des endoparasites des animaux à sang chaud.

16. Composé de formule I selon la revendication 15 pour combattre les nématodes.

17. Composé de formule I selon l'une des revendications 1 à 5 pour combattre des ectoparasites d'animaux ou des parasites de plantes, qu'on applique en quantités de 10 à 1000 g par hectare de surfaces cultivées closes, dans un enclos, des étables et autres espaces.

**Revendications pour l'Etat Contractant AT**

1. Moyen de lutte antiparasitaire contenant outre les véhicules appropriés comme composant actif au moins un dérivé de milbémycine de formule I.

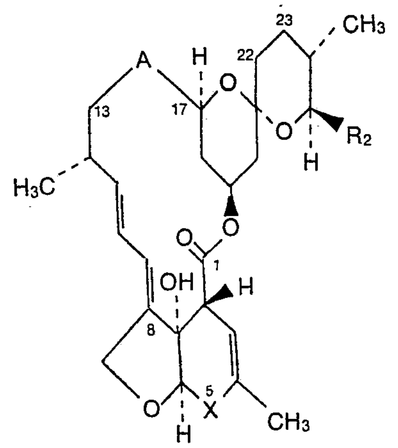

(I),

dans laquelle A est la structure

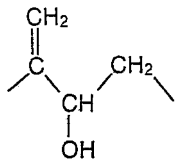

[ = $\Delta^{14,29}$–15–ol]

ou la structure

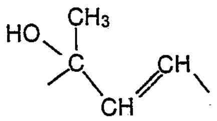

[ = 14-Hydrox –$\Delta^{15,16}$]

tandis que X est soit 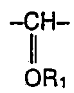 soit 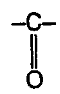

et $R_1$ est l'hydrogène, un groupe silyle de formule

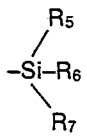

dans lequel $R_5$ est un reste aliphatique $C_1$–$C_4$ ou un benzyle et $R_6$ et $R_7$ représentent indépendamment l'un de l'autre un reste aliphatique $C_1$–$C_4$, benzyle ou phényle ou représente les groupes acyles, $R_3$–CO– ou $R_4$–$SO_2$– dans laquelle $R_3$ est un reste aliphatique $C_1$–$C_6$ non substitué ou halogéné ou un phényle non substitué ou substitué par un alkyle $C_1$–$C_4$ ou un halogène et $R_4$ est un reste alkyle $C_1$–$C_4$ ou un reste phényle non substitué ou substitué par le méthyle, chlore ou nitro;

$R_2$ représente le méthyle, l'éthyle, l'isopropyle ou le sec. butyle.

2. Moyen selon la revendication 1, contenant comme composant actif un composé de formule I, dans laquelle X est soit –CH($OR_1$)– soit –CO–, $R_1$ représente l'hydrogène et $R_2$ représente l'isopropyle.

3. Moyen selon la revendication 1 contenant comme composant actif un composé de formule I, dans laquelle A représente $\Delta^{14,29}$-15-OL.

4. Moyen selon la revendication 3 contenant comme composant actif un composé de formule I, dans laquelle X représente soit –CHOH– soit –CO–.

5. Moyen selon la revendication 3, contenant comme composant actif un composé de formule I dans laquelle $R_1$ représente le groupe silyle cité ci-dessus, dans lequel $R_5$ est le méthyle, le propyle, l'isopropyle, le tert. butyle, et $R_6$ et $R_7$ représentent indépendamment le méthyle, l'éthyle, l'isopropyle, le tert. butyle, le phényle ou le benzyle tandis que $R_2$ est le méthyle, l'éthyle, l'isopropyle ou le sec. butyle.

6. Procédé de préparation de composés de formule I selon la revendication 1, caractérisé en ce qu'on oxyde un composé de formule II

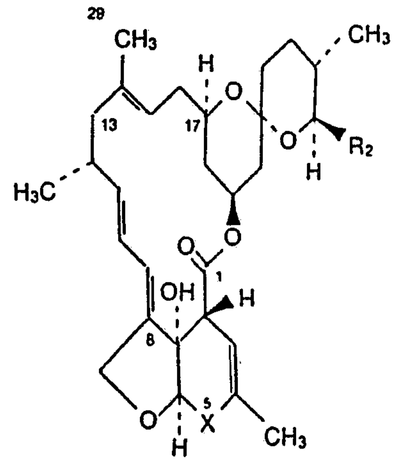

(II),

dans laquelle X et $R_2$ ont la signification donnée pour la formule I, par de l'oxygène radicalaire et qu'on réduit les 15-peroxydes et 14-peroxydes.

7. Procédé selon la revendication 6, dans lequel la réaction est réalisée par la lumière visible en présence d'un sensibilisateur à pression normale dans l'intervalle de température de −90 °C à 45 °C dans un solvant inerte.

8. Procédé selon la revendication 7, dans lequel la réaction est réalisée dans l'intervalle de température de 0 °C à +20 °C.

9. Procédé selon la revendication 6, dans lequel la réaction est réalisée dans un appareil à irradiation avec une lampe de 60–500 watt.

10. Procédé selon la revendication 6, dans lequel l'étape de réduction est réalisée avec $NaBH_4$, $LiALH_4$ ou $P(C_6H_5)_3$.

11. Procédé selon la revendication 6, pour préparer des dérivés de milbémycine D de formule I, dans lesquels A a des significations indiquées, X représente soit –CHOH– soit –CO– et $R_2$ est l'isopropyle, par oxydation par l'oxygène radicalaire de milbémycine D ou de 5-céto-milbémycine D et réduction successive.

12. Moyen selon l'une des revendications 1 à 5 pour combattre des ecto- et endoparasites de plantes et d'animaux.

13. Moyen selon la revendication 12 pour combattre des endoparasites des animaux à sang chaud.

14. Moyen selon la revendication 13 pour combattre les nématodes.

15. Moyen selon l'une des revendications 1 à 5 pour combattre des ectoparasites d'animaux ou des parasites de plantes, qu'on applique en quantités de 10 à 1000 g par hectare de surfaces cultivées closes, dans des enclos, des étables et autres espaces.